# EUROPEAN PATENT SPECIFICATION

(11) **EP 2 240 218 B1**
(45) Date of publication and mention of the grant of the patent: **12.08.2015**
(21) Application number: 08859965.9
(22) Date of filing: 09.12.2008
(51) Int. Cl.: A61M 1/16, A61M 1/34, A61M 39/10

(54) **UNIT FOR THE INFUSION OF A SUBSTITUTE SOLUTION BY A DIALYSIS MACHINE**
EINHEIT ZUR INFUSION EINER ERSATZLÖSUNG MIT EINER DIALYSEMASCHINE
UNITÉ POUR LA PERFUSION D'UNE SOLUTION DE SUBSTITUTION PAR UNE MACHINE DE DIALYSE

(30) Priority: 10.12.2007 IT BO20070811
(43) Date of publication of application: 20.10.2010
(73) Proprietor: Bellco S.r.l., Mirandola (Modena) (IT)
(72) Inventor: CAVANI, Silvia, I-41100 Modena (IT); FIORENZI, Andrea, I-41013 Castelfranco Emilia (IT); FACCHINI, Mauro, I-41037 Mirandola (IT)
(74) Representative: Bosman, Cesare
(86) International application number: PCT/EP2008/067156
(87) International publication number: WO 2009/074588

(56) References cited:
- WO-A-03/041789
- WO-A-03/047656
- US-A- 5 286 067

## Description

### TECHNICAL FIELD

The present invention concerns a unit for the infusion of a substitute solution by a dialysis machine.

### BACKGROUND ART

The haemodiafiltration technique contemplates the purification of the blood by means of the combined action of purification by diffusion and purification by convection. While purification by diffusion consists of balancing the concentrations of two solutions, placed on opposite sides of a semipermeable membrane, purification by convection occurs whenever a pressure gradient is produced between the dialysing solution compartment and the blood compartment and in favour of the latter. In this way, plasma passes through the semipermeable membrane and this movement also determines the passage of the toxic substances dissolved in the plasma. For the convection phenomenon to be efficacious in purifying the blood, the amount of plasma filtered must be high, with the consequence that it is necessary to infuse a saline solution in the blood to maintain a suitable water balance. The saline solution to be infused in the blood is defined here and below as substitute solution.

Substitute solutions are traditionally produced by the pharmaceutical industry in special packs. However, not all packed solutions are stable or can be produced. This is the case, for example, of sodium bicarbonate when it is used as a buffer. An "on line" technique has been known for some time which uses filtration to produce a substitute solution. In other words, the substitute solution is produced directly on the spot by an appliance inside the dialysis machine.

In particular, the substitute solution is produced inside the machine and, by means of a dedicated sterile disposable line, it is infused into the patient in the line that moves his blood either upstream (pre-dilution) or downstream (post-dilution) from the dialysis filter. The disposable line is connected to a special connector corresponding to a front panel of the machine itself.

As may be obvious, the connection of the disposable line to the connector must be able to guarantee as far as possible the absence of contaminations of the substitute solution.

US5286067 discloses a shrouded tubing interface for quickly connecting, disconnecting and avoiding contamination of first and second fluid flow lines comprising male and female connectors, each of which are connected to a respective one of the fluid flow lines.

WO03041789 discloses a connector for forming a sterile connection between two tubes, comprising first and second parts which comprise connectable conduits and which can mate, first and second covers which can be removed from the so-mated parts to expose connectable ends of the first and second conduits, so that the conduits can be connected.

### DISCLOSURE OF INVENTION

The aim of the present invention is to realise a unit for the infusion of a substitute solution by a dialysis machine, the technical characteristics of which are such as to guarantee both the necessary conditions of cleanliness and a convenient and practical use.

The object of this invention is a unit for the infusion of a substitute solution by a dialysis machine whose essential characteristics are reported in claim 1, and whose preferred and/or auxiliary features are set forth in claims 2-5.

### BRIEF DESCRIPTION OF THE DRAWINGS

The following example is given for illustrative purposes without limitation, for a better understanding of the invention with the aid of the figures in the enclosed drawing, wherein:
figure 1 is a perspective view of the connecting device in exploded form;
figure 2 illustrates the connecting device in figure 1 with parts in section;
figure 3 is a perspective view of the unit concerned in the present invention in a flushing conformation;
figure 4 illustrates the unit in figure 3 in section;
figure 5 is a perspective view of the unit concerned in the present invention in a dialysis conformation; and
figure 6 illustrates the unit in figure 5 in section.

### BEST MODE FOR CARRYING OUT THE INVENTION

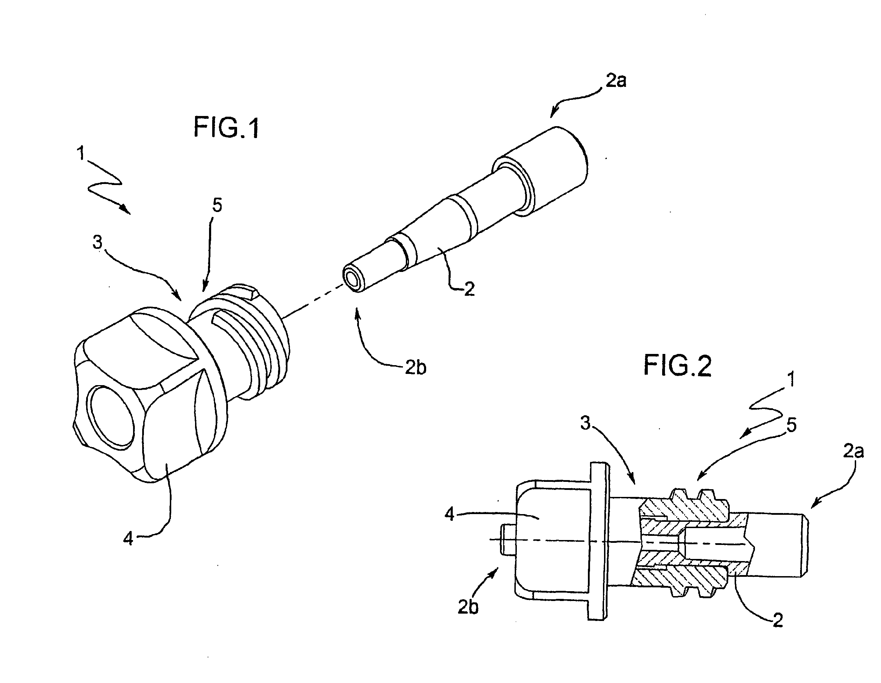

In figures 1 and 2, reference 1 indicates overall a disposable connecting device according to the present invention. The connecting device 1 comprises a tubular element 2 having at a first end a conical female portion 2a for coupling to an internal substitute solution line as will be described below, and at a second end a conical male portion 2b for coupling to a disposable infusion duct. According to a preferred embodiment, the conical male portion 2b is fixed irreversibly to the disposable infusion duct in such a way that the connecting device and the disposable duct itself form a whole disposable infusion line which is sold as such.

The connecting device 1 comprises a ring nut 3 fitted with freedom of rotation around the tubular element 2 to which it is axially restrained. The ring nut 3 comprises a grip 4 to allow personnel to handle it easily, and a threaded portion 5 to allow it to be blocked on a flushing device as described below.

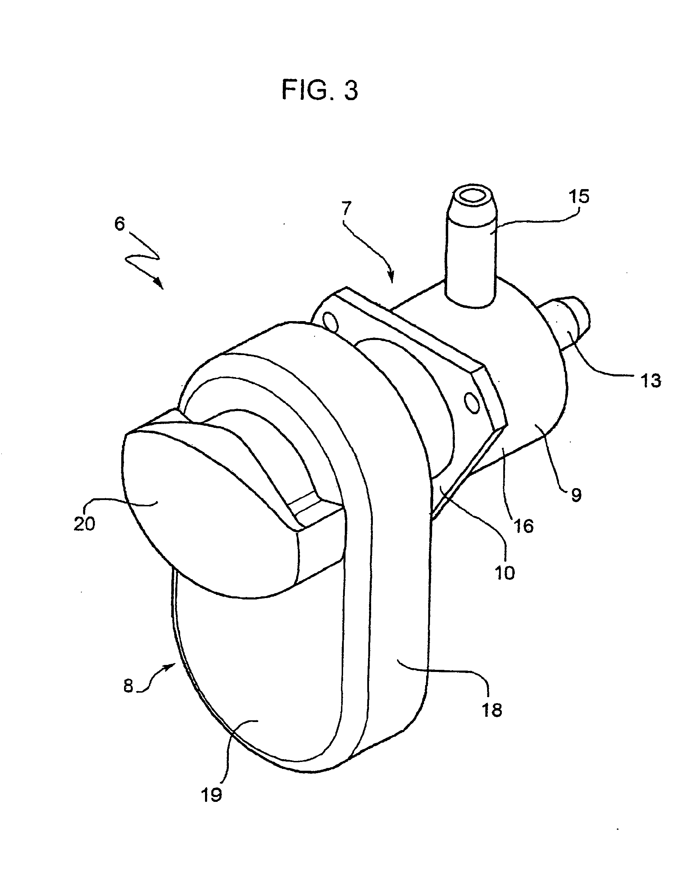

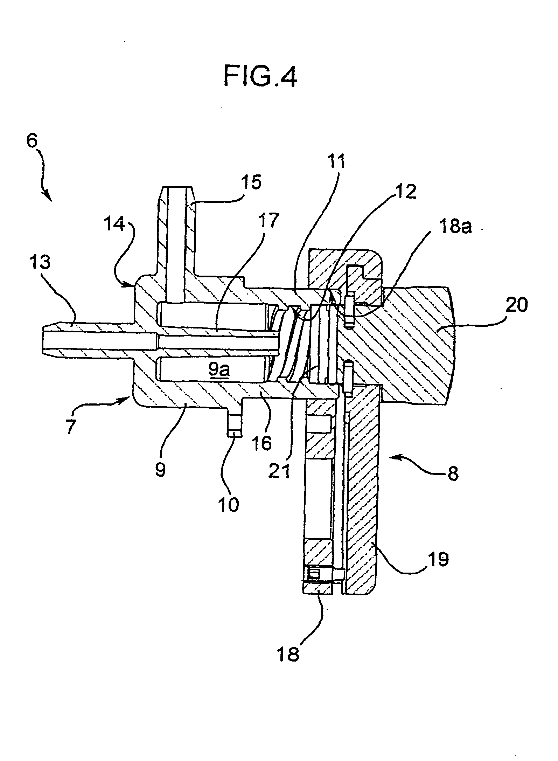

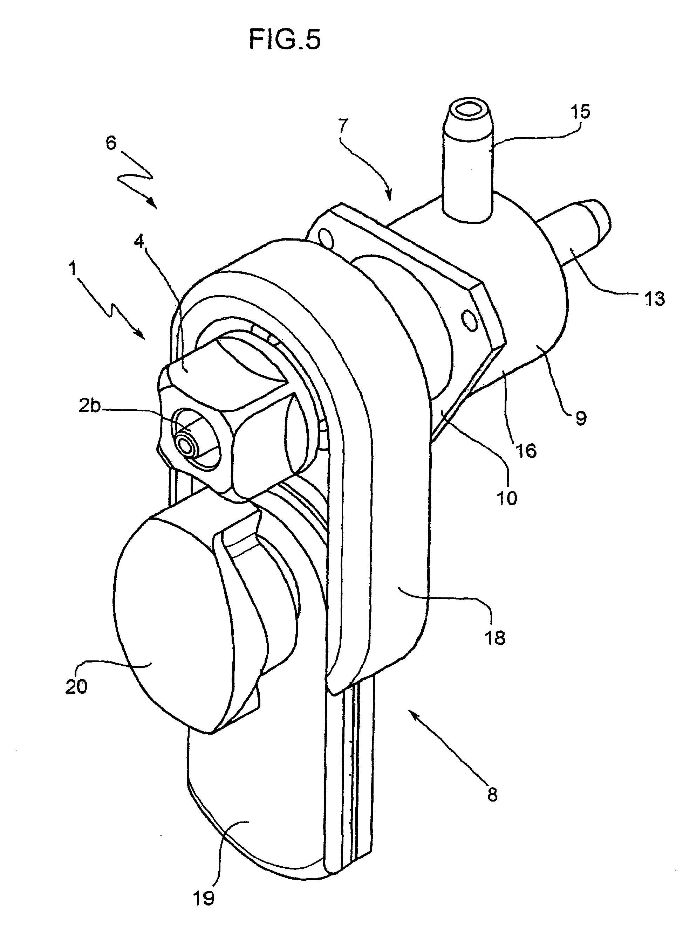

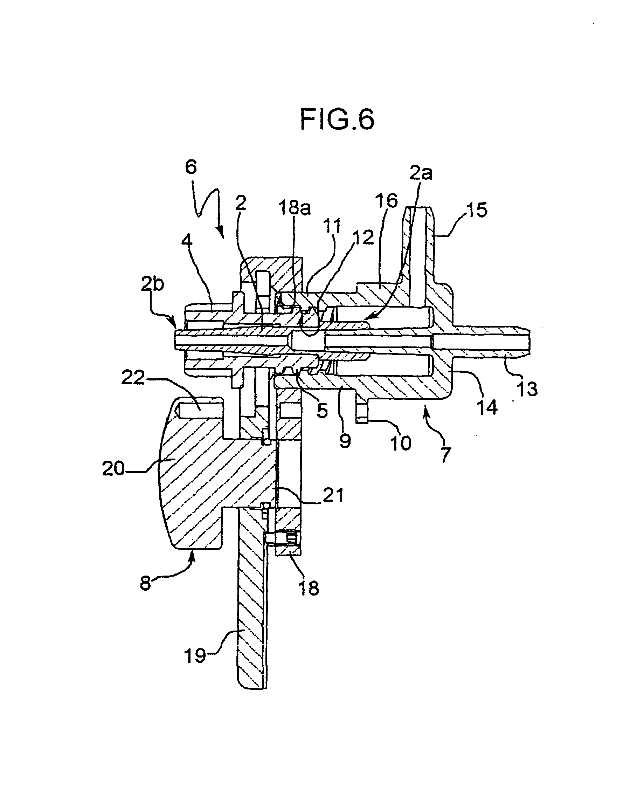

In figures 3 and 4, and in figures 5 and 6 the reference 6 indicates overall the infusion unit concerned in the present invention respectively in a flushing conformation and in a dialysis conformation. The unit 6 comprises a flushing device 7 and a shutter 8 suited to isolate the flushing device 7 if the unit 6 is in a flushing conformation as illustrated in figures 3 and 4.

The flushing device 7 comprises a bowl body 9 defining inside it a flushing chamber 9a. From a side wall 16 of the bowl body 9 there extends radially a flange 10 suited to be fixed onto an internal surface of a front panel of a dialysis machine, not illustrated for reasons of simplicity. The bowl body 9 comprises at an open end a blocking portion 11 with inside it a threaded portion 12.

The flushing device 7 comprises, moreover, a first external connector 13 extending from a back wall 14 of the bowl body 9 and suited to connect with a substitute solution line inside the dialysis machine, a second external connector 15 extending from the side wall 16 of the bowl body 9 and suited to connect with a drainage line, and a connecting cone 17 extending inside the flushing chamber 9a and constituting the hydraulic continuation of the first external connector 13 for the passage of the substitute solution. In particular, as illustrated in figures 5 and 6, the connecting cone 17 is a Luer male cone which, in a dialysis conformation of the unit 6, is coupled to the conical female portion 2a, also Luer type.

The shutter 8 comprises a frame 18 suited to be fixed to an external surface of a front panel of the dialysis machine, a closing slide 19 suited to slide inside the frame 18 engaging a perimeter guide created in the frame 18 itself, and a cap 20. In the frame 18 is created a circular opening 18a, which is engaged by the blocking portion 11. The cap 20 is fitted with freedom of rotation on the closing slide 19 and, in the flushing conformation of the unit 6, it engages in a fluid-tight manner the blocking portion 11 by means of a threaded portion 21. Otherwise, if the unit 6 is in its dialysis conformation (figures 5 and 6), the threaded portion 5 of the ring nut 3 engages the threaded portion 12 of the blocking portion 11.

In use, when the unit 6 is in a flushing conformation (fig. 3 and 4), the closing slide 19 is in a raised position and the cap 20 engages the blocking portion 11 as described above, thus isolating the flushing chamber 9a. In this conformation a flushing fluid (water/disinfectant) is circulated in the first external connector 13 and in the connecting cone 17, and enters the flushing chamber 9a from which it comes out through the second external connector 15 to be conveyed into a drainage line. As shown by figure 4, in the flushing conformation the connecting cone 17 is completely immersed in the flushing fluid thus ensuring it is clean and disinfected.

Instead, when the unit 6 is in a dialysis conformation (fig. 5 and 6), the closing slide 19 is in a lowered position and the cap 20 no longer engages the blocking portion 11, which is instead engaged by the connecting device 1. In particular, the conical female portion 2a of the connecting element 2 couples with the connecting cone 17, within which passes the substitute solution which, through the conical male portion 2b, is sent into a disposable duct (known and not described for reasons of simplicity) of the disposable line to which the connecting device 1 also belongs.

The blocking and correct inserting of the connecting device 1 in the flushing device 7 is due to the reciprocal engagement with the threaded portion 12 of the blocking portion 11. By means of the grip 4 the user turns the ring nut 3, realising the reciprocal engagement of the above-mentioned threaded portions 5 and 12. The main advantage lies in the fact that, with the connecting device 1 according to the present invention, it is possible to turn the ring nut without at the same time twisting the disposable duct fixed to the conical male portion 2b. In fact, as specified above, the ring nut 3 is fitted with freedom of rotation around the connecting element 2.

Lastly, on the cap 20 is fitted a position magnet 22 which interfaces with a position reader (not illustrated) fixed on the frame 17 in such a way that, when the cap 20 is turned to disengage the blocking portion 11 and allow the connecting device 1 to be inserted in it, the reader notices the shift of the magnet and commands the automatic interruption of the flow of fluid.

Further advantages lie in the technical characteristics of the shutter 8, which by means of the guide and slide coupling of the closing slide 19 with the frame 18, guarantees a reduced bulk and at the same time a constant control of the position of the cap 20 even when the unit 6 is in its dialysis conformation.

Lastly, the technical characteristics of the connecting device 1 allow there to be a grip 4 positioned distant from the conical female portion 2a so as to guarantee further the required conditions of cleanliness.

## Claims

1. Unit (6) for the infusion of a substitute solution by a dialysis machine; said unit (6) comprising a flushing device (7) internally defining a flushing chamber (9a) and comprising a connecting cone (17) extending inside the flushing chamber (9a) and suited to be hydraulically connected to a substitute solution line inside a dialysis machine; a connecting device (1) suited to connect said connecting cone (17) to a duct for infusing the substitute solution to a patient; and a shutter element (8) suited to close the flushing device (7) to isolate said flushing chamber (9a) if the unit (6) is in a flushing conformation; wherein said connecting device (1) comprises a tubular element (2) having a first joining end (2a) suited to be connected to said connecting cone (17) of said flushing device (7) and a second joining end (2b) suited to be connected to an infusion duct; and a blocking ring nut (3) housed with freedom of rotation around the tubular element (2) to which it is axially restrained and structured to engage and block said flushing device (7) if the connecting unit (6) is in a dialysis conformation; said blocking ring nut (3) comprising a grip (4) and a threaded portion (5) suited to engage a threaded portion (12) of said flushing device (7).

2. Infusion unit according to claim 1, **characterised in that** said coupling cone (17) of said flushing device (7) and said first joining end (2a) of the tubular element (2) are respectively a male Luer cone and a female Luer cone.

3. Infusion unit according to one of the preceding claims, **characterised in that** said shutter (8) comprises a frame (18) suited to be fixed to an external surface of a front panel of the dialysis machine, a closing slide (19) suited to slide inside the frame (18), and a cap (20) fitted with freedom of rotation on the closing slide (19) and suited to close said flushing chamber (9a) when the unit (6) is in flushing conformation.

4. Infusion unit according to claim 3, **characterised in that** said shutter element comprises a position magnet (22) fitted on the cap (20) and a position reader fixed on the frame (17) and such as to notice the shift of the magnet (22) and command the automatic interruption of the flow of fluid.

5. Infusion unit according to one of the preceding claims, **characterised in that** said connecting device (1) is fixed irreversibly to a disposable infusion duct to form a whole disposable infusion line.

## Patentansprüche

1. Einheit (6) zur Infusion einer Substitutionslösung durch eine Dialysemaschine; wobei die Einheit (6) Folgendes aufweist: eine Spülvorrichtung (7), die innen eine Spülkammer (9a) definiert, und die einen Verbindungskonus (17) aufweist, der sich innerhalb der Spülkammer (9a) erstreckt und geeignet ist, um mit einer Substitutionslösungsleitung innerhalb einer Dialysemaschine hydraulisch verbunden zu werden; eine Verbindungsvorrichtung (1), die geeignet ist, um den Verbindungskonus (17) mit einer Leitung zum Infundieren der Substitutionslösung in einen Patienten zu verbinden; und ein Verschlusselement (8), das geeignet ist, um die Spülvorrichtung (7) zu verschließen, um die Spülkammer (9a) zu isolieren, wenn sich die Einheit (6) in einer Spül-Stellung befindet;
wobei die Verbindungsvorrichtung (1) ein röhrenförmiges Element (2) mit einem ersten Verbindungsende (2a) aufweist, welches geeignet ist, um mit dem Verbindungskonus (17) der Spülvorrichtung (7) verbunden zu werden, und ein zweites Verbindungsende (2b), welches geeignet ist, um mit einer Infusionsleitung verbunden zu werden; und eine Sperrschraubmutter (3), die mit einem Drehungsfreiheitsgrad um das röhrenförmige Element (2) herum aufgenommen ist, an dem sie axial zurückgehalten wird und aufgebaut ist, um mit der Spülvorrichtung (7) in Eingriff zu kommen und diese zu blockieren, wenn sich die Verbindungseinheit (6) in einer Dialyse-Stellung befindet, wobei die Sperrschraubmutter (3) einen Handgriff (4) und einen mit Gewinde versehenen Teil (5) aufweist, der geeignet ist, um mit einem mit Gewinde versehenen Teil (12) der Spülvorrichtung (7) in Eingriff zu kommen.

2. Infusionseinheit nach Anspruch 1, **dadurch gekennzeichnet, dass** der Kupplungskonus (17) der Spülvorrichtung (7) und das erste Verbindungsende (2a) des röhrenförmigen Elementes (2) jeweils ein Luer-Konus-Stecker und eine Luer-Konus-Buchse sind.

3. Infusionseinheit nach einem der vorhergehenden Ansprüche, **dadurch gekennzeichnet, dass** das Verschlusselement (8) Folgendes aufweist: einen Rahmen (18), der geeignet ist, um an einer Außenfläche einer Frontplatte der Dialysemaschine befestigt zu werden, einen Schließschieber (19), der geeignet ist, innerhalb des Rahmens (18) zu gleiten, und eine Kappe (20), die mit einem Drehfreiheitgrad auf dem Schließschieber (19) montiert ist und geeignet ist, um die Spülkammer (9a) zu verschließen, wenn die Einheit (6) in der Spül-Stellung ist.

4. Infusionseinheit nach Anspruch 3, **dadurch gekennzeichnet, dass** das Verschlusselement einen Positionsmagneten (22) aufweist, der auf der Kappe (20) montiert ist, und einen Positionsleser, der auf dem Rahmen (17) befestigt ist, und zwar so, dass er die Verschiebung des Magneten (22) wahrnimmt und die automatische Unterbrechung des Strömungsmittelflusses anordnet.

5. Infusionseinheit nach einem der vorhergehenden Ansprüche, **dadurch gekennzeichnet, dass** die Verbindungsvorrichtung (1) irreversibel an einer Einweginfusionsleitung befestigt ist, um einen komplette Einweginfusionsleitungsstrang zu bilden.

## Revendications

1. Unité (6) pour la perfusion d'une solution de substitution par une machine de dialyse ; ladite unité (6) comprenant un dispositif de rinçage (7) définissant intérieurement, une chambre de rinçage (9a) et comprenant un cône de raccordement (17) s'étendant à l'intérieur de la chambre de rinçage (9a) et approprié pour être raccordé, hydrauliquement, à une ligne de solution de substitution à l'intérieur d'une machine de dialyse ; un dispositif de raccordement (1) approprié pour raccorder ledit cône de raccordement (17) à un conduit pour perfuser la solution de substitution à un patient ; et un élément d'obturation (8) approprié pour fermer le dispositif de rinçage (7) afin d'isoler ladite de chambre de rinçage (9a) si l'unité (6) est dans une conformation de rinçage ; dans laquelle ledit dispositif de raccordement (1) comprend un élément tubulaire (2) ayant une première extrémité d'assemblage (2a) appropriée pour être raccordée audit cône de raccordement (17) dudit dispositif de rinçage (7) et une seconde extrémité d'assemblage (2b) appropriée pour être raccordée à un conduit de perfusion ; et un écrou annulaire de blocage (3) logé avec une liberté de rotation autour de l'élément tubulaire (2) sur lequel il est axialement retenu et structuré pour mettre en prise et bloquer ledit dispositif de rinçage (7) si l'unité de raccordement (6) est dans une conformation de dialyse ; ledit écrou annulaire de blocage (3) comprend une prise (4) et une partie filetée (5) appropriée pour mettre en prise une partie filetée (12) dudit dispositif de rinçage (7).

2. Unité de perfusion selon la revendication 1, **caractérisée en ce que** ledit cône de couplage (17) dudit dispositif de rinçage (7) et ladite première extrémité d'assemblage (2a) de l'élément tubulaire (2) sont respectivement un cône Luer mâle et un cône Luer femelle.

3. Unité de perfusion selon l'une des revendications précédentes, **caractérisée en ce que** ledit obturateur (8) comprend un bâti (18) approprié pour être fixé sur une surface externe d'un panneau avant de la machine de dialyse, une glissière de fermeture (19) appropriée pour coulisser à l'intérieur du bâti (18), et un capuchon (20) monté avec une liberté de rotation sur la glissière de fermeture (19) et approprié pour fermer ladite chambre de rinçage (9a) lorsque l'unité (6) est en conformation de rinçage.

4. Unité de perfusion selon la revendication 3, **caractérisée en ce que** ledit élément d'obturateur comprend un aimant de positionnement (22) monté sur le capuchon (20) et un lecteur de position fixé sur le bâti (17) et afin de notifier le déplacement de l'aimant (22) et de commander l'interruption automatique de l'écoulement de fluide.

5. Unité de perfusion selon l'une des revendications précédentes, **caractérisée en ce que** le dispositif de raccordement (1) est fixé, de manière irréversible, sur un conduit de perfusion jetable afin de former une ligne de perfusion jetable complète.
